(19) Europäisches Patentamt
European Patent Office
Office européen des brevets

(11) Veröffentlichungsnummer: **0 116 123**
**B1**

(12) # EUROPÄISCHE PATENTSCHRIFT

(45) Veröffentlichungstag der Patentschrift: **15.10.86**

(51) Int. Cl.⁴: **C 08 F 2/50**, G 03 C 1/68

(21) Anmeldenummer: **83110789.1**

(22) Anmeldetag: **28.10.83**

(54) **Verfahren zur Photopolymerisation von Vinylverbindungen und photopolymerisierbares Material (I).**

(30) Priorität: **14.01.83 DE 3301010**

(43) Veröffentlichungstag der Anmeldung: **22.08.84 Patentblatt 84/34**

(45) Bekanntmachung des Hinweises auf die Patenterteilung: **15.10.86 Patentblatt 86/42**

(84) Benannte Vertragsstaaten: **AT CH DE FR GB LI SE**

(56) Entgegenhaltungen: **FR-A-2 273 042**
**GB-A-1 529 863**

(73) Patentinhaber: **Kulzer & Co. GmbH, Philipp- Reis-Strasse 8, D-6393 Wehrheim (TS.)1 (DE)**

(72) Erfinder: **Schaefer, Roland, Dr., Merianweg 5, D-6382 Friedrichsdorf (DE)**

(74) Vertreter: **Heinen, Gerhard, Dr., Heraeusstrasse 12-14, D-6450 Hanau/Main (DE)**

EP 0 116 123 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Photopolymerisation von Vinylverbindungen in Gegenwart eines Photoinitiators aus

a) mindestens einem Photosensibilisator der allgemeinen Formel

$$A - \underset{\underset{O}{\|}}{C} - (X)_n - A$$

mit X = CO, C($R^1$)($R^2$) oder C($R^3$)($OR^4$), worin $R^1$, $R^2$, $R^3$ und $R^4$ H oder Kohlenwasserstoff-Reste bedeuten,

n = 0 oder 1,

A = gegebenenfalls substituierte Kohlenwasserstoff-Reste, die miteinander verbunden sein können und für n = 1 und X = C($R^1$)($R^2$) und für n = 0 aromatische Reste sind, und

b) mindestens einem Reduktionsmittel und photopolymerisierbaren Materialien.

Die Photopolymerisation findet vielseitige, auch technische Anwendung, zum Beispiel zur Härtung von Lacken und Überzügen, zur Herstellung von Druckplatten und beim Buchdruck.

Auch auf dem Dentalgebiet wird die Photopolymerisation angewandt. Photopolymerisierbare Materialien dienen zur Herstellung von Zahnfüllungen und -versiegelungen, von Kronen und Brücken und von künstlichen Gebissen (siehe zum Beispiel die britische Patentschrift 569 974 und die deutschen Offenlegungs- beziehungsweise Auslegeschriften 23 15 645, 23 57 324, 29 10 077 und 29 14 537).

In der britischen Patentschrift 1 408 265 bzw. 1 529 863 werden photopolymerisierbare Materialien beschrieben, die als Photoinitiator ein Gemisch aus

a) mindestens einem Photosensibilisator der allgemeinen Formel

$$A - \underset{\underset{O}{\|}}{C} - (X)_n - A$$

mit X = CO, C($R^1$) ($R^2$) oder C($R^3$) ($OR^4$), worin $R^1$, $R^2$, $R^3$ und $R^4$ H oder Kohlenwasserstoff-Reste bedeuten,

n = 0 oder 1,

A = gegebenenfalls substituierte Kohlenwasserstoff-Reste, die miteinander verbunden sein können und für n = 1 und X = C($R^1$) ($R^2$) und für n = 0 aromotische Reste sind, und

b) mindestens einem Reduktionsmittel der allgemeinen Formel

$$R - \overset{\overset{R}{|}}{M} - R,$$

worin M ein Element der Gruppe VB bedeutet, das gegebenenfalls mit zwei Resten R einen Ring bilden kann, und mindestens einer der Reste R in α-Stellung zu M eine CH-Gruppe aufweisen muß, wenn ein anderer eine aromatische Gruppe ist,

enthalten und durch Bestrahlung mit sichtbarem Licht oder durch UV-Strahlen ausgehärtet werden können.

Als Beispiele für die Photosensibilisatoren werden u. a. Biacetyl, Benzil, p,p'-Dialkoxybenzil, Benzoin und Campherchinon, für die Reduktionsmittel Propylamin, Dimethylaminoäthylmethacrylat, N,N'-Dimethylanilin und Piperidin genannt.

Aus der deutschen Patentanmeldung DE-A-31 36 484 ist ein Verfahren zur Photopolymerisation von Vinylverbindungen in Gegenwart von Ketonen als Photosensibilisatoren und von cyclischen beziehungsweise heterocyclischen Verbindungen, besonders von 5-substuierten Barbitursäuren, als Reduktionsmittel bekannt.

Es ist die Aufgabe der Erfindung, ein Verfahren zur Photopolymerisation von Vinylverbindungen in der Art des in der GB-PS 1 408 265 beschriebenen zu finden, das ein rasches Aushärten der Vinylverbindungen bewirkt. Die nach dem Verfahren hergestellten Polymerisate sollen eine gute Farbbeständigkeit besitzen.

Das die Lösung der Aufgabe darstellende Verfahren ist erfindungsgemäß dadurch gekennzeichnet, daß als Reduktionsmittel ein 1-Aryl-2,5-dialkylpyrrol verwendet wird.

Besonders bewährt hat sich das erfindungsgemäße Verfahren, wenn als Photosensibilisator ein Benzoinalkyläther, zum Beispiel Benzoinmethyläther, oder ein α-Diketon, vorzugsweise Benzil oder Campherchinon, verwendet wird. Es kann vorteilhaft sein, anstelle der beiden zuletzt genannten Photosensibilisatoren oder daneben ein Benzildialkylketal zu verwenden. Ein besonders geeignetes Photosensibilisator-Gemisch ist das aus Campherchinon und Benzildimethylketal.

Es war überraschend, daß die Photopolymerisation in Gegenwart von Carbonylverbindungen durch in α-Stellung keine CH-Gruppen aufweisende N-Aryl-Amine in höherem Maße als durch aus der GB-PS 1 408 265 bekannte N-Aryl-Amine beschleunigt wird.

Die nach dem erfindungsgemäßen Verfahren erhaltenen Vinylpolymeren und -copolymeren weisen eine sehr gute Farbbeständigkeit auf.

Das erfindungsgemäße Verfahren kann überall dort angewandt werden, wo monomere Vinylverbindungen beziehungsweise solche

Verbindungen enthaltende Materialien durch Bestrahlen mit UV-Licht oder mit sichtbarem Licht polymerisiert werden sollen.

Unter Vinylverbindungen werden alle gebräuchlichen äthylenisch ungesättigten Verbindungen verstanden, besonders Acryl- und Methacrylsäureester ein- und mehrwertiger Alkohole, darunter auch die sogenannten Urethanacrylate und -methacrylate und das aus der US-PS 3 066 112 bekannte Bis-GMA, das Reaktionsprodukt aus Bis-Phenol A und Glycidylmethacrylat.

Den Vinylverbindungen beziehungsweise den diese Vinylverbindungen enthaltenden Materialien wird der Photosensibilisator zweckmäßigerweise in einer Menge von $10^{-2}$ bis 10 Gewichts-%, bezogen auf die Vinylverbindungen, zugesetzt; bevorzugt wird eine Menge von $10^{-1}$ bis 5 Gewichts-%. Das Reduktionsmittel kann in ebensolchen Mengen eingesetzt werden.

Besonders bewährt hat sich die Anwendung des erfindungsgemäßen Verfahrens auf dem Dentalgebiet für die Herstellung sowohl von Zahnfüllungen und -versiegelungen als auch von Kronen, Brücken und künstlichen Gebissen durch Polymerisation der Acrylsäure- und/oder Methacrylsäureester und gegebenenfalls anorganische Füllstoffe enthaltenden Materialien unter Bestrahlen mit UV-Licht oder mit sichtbarem Licht.

In den folgenden Beispielen werden Methacrylsäureester und anorganische Füllstoffe enthaltende photopolymerisierbare Materialien und deren Polymerisation gemäß der Erfindung durch Bestrahlen mit UV-Licht (Beispiele 1 bis 3) bezichungsweise mit sichtbarem Licht (Beispiele 4 bis 8) beschrieben.

Die Polymerisation von photopolymerisierbarem Material, das als Reduktionsmittel N-Aryl-N-Verbindungen mit CH-Gruppen in Nachbarstellung zum Stickstoff-Atom enthält, wird im Vergleich zu den Beispielen gemäß der Erfindung in den Beispielen 9 bis 12 beschrieben.

Die Schichtdicke der erhaltenen polymeren Formkörper wird gemessen; sie dient zur Beurteilung beziehungsweise zum Vergleich der Photoinitiator-Aktivität.

**Beispiele 1 bis 3**

(Polymerisation durch Bestrahlen mit UV-Licht)

Eine Mischung aus

7,0 g Bis-GMA,

3,0 g Triäthylenglykoldimethacrylat,

30,0 g Lithiumaluminiumsilicat (85 Gewichts-% der Teilchen mit Teilchengröße < 15 µm),

1,0 g Aluminiumoxid und

X Photoinitiator (siehe Tabelle 1)

wird in eine Form (Innendurchmesser 6 mm, Höhe 10 mm) aus Delrin®, ein Polyacetal-Kunststoff, gegeben, an der Oberfläche mit Polyesterfolie abgedeckt und 20 Sekunden lang mit dem UV-Polymerisationsgerät Duralux der Firma Kulzer, Duralux UV ZO A, in der Weise bestrahlt, daß das Lichtaustrittsfenster des Gerätes auf die Polyesterfolie gesetzt wird.

Dann wird der unpolymerisiert gebliebene Teil der Mischung entfernt und die Schichtdicke des polymerisierten Teils gemessen.

Art und Menge des Photoinitiators und die Schichtdicke werden in der Tabelle 1 angegeben.

**Tabelle 1**

| Beispiel | Photoinitiator | Gewichts-% | Schichtdicke [mm] |
|---|---|---|---|
| 1 | Benzoinmethyläther + 1-(3,4-Dimethylphenyl)-2,5-dimethylpyrrol | 0,1 / 0,1 | 2,2 |
| 2 | Benzil + 1-(3,4-Dimethylphenyl)-2,5-dimethylpyrrol | 0,1 / 0,1 | 1,7 |
| 3 | Benzildimethylketal + 1-(3,4-Dimethylphenyl)-2,5-dimethylpyrrol | 0,3 / 0,1 | 2,5 |

**Beispiele 4 bis 8**

(Polymerisation durch Bestrahlen mit sichtbarem Licht)
Eine Mischung aus
7,0 g Bis-GMA,
3,0 g Triäthylenglykoldimethacrylat,
30,0 g Lithiumaluminiumsilicat (85 Gewichts-% der Teilchen mit Teilchengröße < 15 µm),
1,0 g Aluminiumoxid und
X Photoinitiator (siehe Tabelle 2)
wird in eine Form (Innendurchmesser 6 mm, Höhe 10 mm) aus Delrin®, ein Polyacetal-Kunststoff, gegeben, an der Oberfläche mit Polyesterfolie abgedeckt und 20 Sekunden lang mit dem Wolfram-Halogen-Lichtgerät Translux der Firma Kulzer in der Weise bestrahlt, daß das Lichtaustrittsfenster des Gerätes auf die Polyesterfolie gesetzt wird.

Dann wird der unpolymerisiert gebliebene Teil der Mischung entfernt und die Schichtdicke des polymerisierten Teils gemessen.

Art und Menge des Photoinitiators und die Schichtdicke werden in der Tabelle 2 angegeben.

**Tabelle 2**

| Beispiel | Photoinitiator | Gewichts-% | Schichtdicke [mm] |
|---|---|---|---|
| 4 | Campherchinon + 1-(3,4-Dimethylphenyl)-2,5-dimethylpyrrol | 0,1<br>0,1 | 7,5 |
| 5 | Campherchinon + Benzildimethylketal + 1-(3,4-Dimethylphenyl)-2,5-dimethylpyrrol | 0,1<br>0,3<br>0,1 | 8,2 |
| 6 | Campherchinon + Benzildimethylketal + 1-(4-Äthoxycarbonylphenyl)-2,5-dimethylpyrrol | 0,1<br>0,3<br>0,1 | 7,1 |
| 7 | Campherchinon + Benzildimethylketal + 1-(2,4,6-Trimethylphenyl)-2,5-dimethylpyrrol | 0,1<br>0,3<br>0,1 | 8,2 |
| 8 | Campherchinon + Benzildimethylketal + p,p'-Di-(2,5-dimethylpyrrolyl)-diphenyl-sulfon | 0,1<br>0,3<br>0,1 | 6,1 |

**Vergleichsbeispiele 9 bis 12**

(Polymerisation durch Bestrahlen mit sichtbarem Licht)
Eine Mischung aus
7,0 g Bis-GMA,
3,0 g Triäthylenglykoldimethacrylat,
30,0 g Lithiumaluminiumsilicat (85 Gewichts-% der Teilchen mit Teilchengröße < 15 µm),
1,0 g Aluminiumoxid und
X Photoinitiator (siehe Tabelle 3)
wird in eine Form (Innendurchmesser 6 mm, Höhe 10 mm) aus Delrin®, ein Polyacetal-Kunststoff, gegeben, an der Oberfläche mit Polyesterfolie abgedeckt und 20 Sekunden lang mit dem Wolfram-Halogen-Lichtgerät Translux der Firma Kulzer in der Weise bestrahlt, daß das Lichtaustrittsfenster des Gerätes auf die Polyesterfolie gesetzt wird.

Dann wird der unpolymerisiert gebliebene Teil der Mischung entfernt und die Schichtdicke des polymerisierten Teils gemessen.

Art und Menge des Photoinitiators und die Schichtdicke werden in der Tabelle 3 angegeben.

Benzildialkylketal = 2,2-Dialkoxy-1,2-diphenyläthanon

Benzildimethylketal = 2,2-Dimethoxy-1,2-diphenyläthanon

**Tabelle 3 (Vergleichsversuche)**

| Beispiel | Photoinitiator | Gewichts-% | Schichtdicke [mm] |
|---|---|---|---|
| 9 | Campherchinon<br>+<br>Benzildimethylketal<br>+<br>N,N-Dihydroxyäthyl-p-toluidin | 0,1<br><br>0,3<br><br>0,1 | 4,7 |
| 10 | Campherchinon<br>+<br>Benzildimethylketal<br>+<br>1-Phenylpyrrol | 0,1<br><br>0,3<br><br>0,1 | 4,8 |
| 11 | Campherchinon<br>+<br>Benzildimethylketal<br>+<br>1-Methylindol | 0,1<br><br>0,3<br><br>0,1 | 3,7 |
| 12 | Campherchinon<br>+<br>Benzildimethylketal<br>+<br>10-Methyl-9(10H)-acridon | 0,1<br><br>0,3<br><br>0,1 | 4,7 |

**Patentansprüche**

1. Verfahren zur Photopolymerisation von Vinylverbindungen in Gegenwart eines Photoinitiators aus

a) mindestens einem Photosensibilisator der allgemeinen Formel

$$A - \underset{\underset{O}{\|}}{C} - (X)_n - A$$

mit X = CO, $C(R^1)(R^2)$ oder $C(R^3)(OR^4)$, worin $R^1$, $R^2$, $R^3$ und $R^4$ H oder Kohlenwasserstoff-Reste bedeuten,

n = 0 oder 1,

A = gegebenenfalls substituierte Kohlenwasserstoff-Reste, die miteinander verbunden sein können und für n = 1 und X = $C(R^1)(R^2)$ und für n = 0 aromatische Reste sind, und

b) mindestens einem Reduktionsmittel,

dadurch gekennzeichnet, daß als Reduktionsmittel ein 1-Aryl-2,5-dialkylpyrrol verwendet wird.

2. Verfahren nach Anspruch 1, dadurch gekennzeichnet, daß als Reduktionsmittel 1-(3,4-Dimethylphenyl)-2,5-dimethyl-pyrrol oder 1-(4-äthoxycarbonylphenyl)-2,5-dimethylpyrrol verwendet wird.

3. Verfahren nach Anspruch 1 oder 2, dadurch gekennzeichnet, daß als Photosensibilisator ein Benzoinalkyläther verwendet wird.

4. Verfahren nach Anspruch 1 oder 2, dadurch gekennzeichnet, daß als Photosensibilisator Benzil verwendet wird.

5. Verfahren nach Anspruch 1 oder 2, dadurch gekennzeichnet, daß als Photosensibilisator Campherchinon verwendet wird.

6. Verfahren nach Anspruch 5, dadurch gekennzeichnet, daß anstelle des Campherchinons oder daneben ein Benzildialkylketal verwendet wird.

7. Photopolymerisierbares Material, das mindestens eine Vinylverbindung und einen Photoinitiator aus

a) mindestens einem Photosensibilisator der allgemeinen Formel

$$A - \underset{\underset{O}{\|}}{C} - (X)_n - A$$

mit X = CO, $C(R^1)(R^2)$ oder $C(R^3)(OR^4)$, worin $R^1$, $R^2$, $R^3$ und $R^4$ H oder Kohlenwasserstoff-Reste bedeuten,

n = 0 oder 1,
A = gegebenenfalls substituierte Kohlenwasserstoff-Reste, die miteinander verbunden sein können und für n = 1 und X = $C(R^1)(R^2)$ und für n = 0 aromatische Reste sind,
und
b) mindestens einem Reduktionsmittel enthält, dadurch gekennzeichnet, daß das Reduktionsmittel ein 1-Aryl-2,5-dialkylpyrrol ist.

8. Photopolymerisierbares Material nach Anspruch 7, dadurch gekennzeichnet, daß der Photosensibilisator Benzil oder Campherchinon ist.

9. Photopolymerisierbares Material nach Anspruch 8, dadurch gekennzeichnet, daß der Photosensibilisator aus Campherchinon und einem Benzildialkylketal besteht.

10. Photopolymerisierbares Material nach Anspruch 9, dadurch gekennzeichnet, daß es als Vinylverbindung Acrylsäureester und/oder Methacrylsäureester enthält.

**Claims**

1. A process for the photopolymerization of vinyl compounds in the presence of a photoinitiator consisting of
a) at least one photosensitizer of the general formula

$$A - \underset{\underset{O}{\|}}{C} - (X)_n - A$$

with X = CO, $C(R^1)(R^2)$ or $C(R^3)(OR^4)$, in which $R^1$, $R^2$, $R^3$
and $R^4$ denote H or hydrocarbon residues,
n = 0 or 1,
A = possibly substituted hydrocarbon residues which can be connected to one another and for n = 1 and X = $C(R^1)(R^2)$ and for n = 0 are aromatic residues,
and
b) at least one reducing agent,
characterised in that a l-Aryl-2,5-dialkylpyrrole is used as reducing agent.

2. A process according to claim 1, characterised in that 1-(3,4-Dimethylphenyl)-2,5-dimethylpyrrole or 1-(4-Ethoxycarbonylphenyl)-2,5-dimethylpyrrole is used as a reducing agent.

3. A process according to claim 1 or 2, characterised in that a benzoin alkylether is used as a photosensitizer.

4. A process according to claim 1 or 2, characterised in that benzil is used as a photosensitizer.

5. A process according to claim 1 or 2, characterised in that camphor quinone is used as a photosensitizer.

6. A process according to claim 5, characterised in that, instead of the camphor quinone or in addition thereto, a benzildialkylketal is used.

7. Photopolymerizable material, which contains at least one vinyl compound and a photoinitiator consisting of
a) at least one photosensitizer of the general formula A-

$$A - \underset{\underset{O}{\|}}{C} - (X)_n - A$$

with X = CO, $C(R^1)(R^2)$ or $C(R^3)(OR^4)$, in which $R^1$, $R^2$, $R^3$ and
$R^4$ denote H or hydrocarbon residues,
n = 0 or 1,
A = possibly substituted hydrocarbon residues which can be connected to one another and for n = 1 and
X = $C(R^1)(R^2)$ and for n = 0 are aromatic residues,
and
b) at least one reducing agent,
characterised in that the reducing agent is a 1-Aryl-2,5-dialkylpyrrole.

8. Photopolymerizable material according to claim 7, characterised in that the photosensitizer is benzil or camphor quinone.

9. Photopolymerizable material according to claim 8, characterised in that the photosensitizer consists of, camphor quinone and a, benzildialkylketal.

10. Photopolymerizable material according to claim 9, characterised in that it contains am acrylic acid ester and/or a methacrylic acid ester as the vinyl compound.

**Revendications**

1. Procédé pour la photopolymérisation de composés vinyliques en présence d'un photo-inducteur consistant en:
a) au moins un photosensibilisateur de formule générale

$$A - \underset{\underset{O}{\|}}{C} - (X)_n - A$$

dans laquelle
X = CO, $C(R^1)(R^2)$ ou $C(R^3)(OR^4)$, ou $R^1$, $R^2$, $R^3$ et $R^4$ représentent H ou des restes d'hydrocarbures,
n = 0 ou 1,

A = des restes d'hydrocarbures éventuellement substitués qui peuvent être reliés entre eux et, pour n = 1 et X = $C(R^1)(R^2)$ et pour n = 0, des restes aromatiques,

b) au moins un agent réducteur,

caractérisé en ce que l'on utilise comme agent réducteur un 1-aryl-2,5-dielkylpyrrole.

2. Procédé selon la revendication 1, caractérisé en ce que l'on utilise comme agent réducteur le 1-(3,4-diméthylphényl)-2,5-diméthylpyrrole ou le 1-(4-éthoxycarbonylphényl)-2,5-diméthyl-pyrrole.

3. Procédé selon la revendication 1 ou 2, caractérisé en ce que l'on utilise comme photosensibilisateur un éther d'alkyle de la benzoïne.

4. Procédé selon la revendication 1 ou 2, caractérisé en ce que l'on utilise comme photosensibilisateur le benzile.

5. Procédé selon la revendication 1 ou 2, caractérisé en ce que l'on utilise comme photosensibilisateur la camphoquinone.

6. Procédé selon la revendication 5, caractérisé en ce que l'on utilise un dialkylacétal du benzile à la place ou en plus de la camphoquinone.

7. Matériau photopolymérisable qui contient au moins un composé vinylique et un photo-inducteur consistant en:

a) au moins un photosensibilisateur de formule générale

$$A - \underset{\underset{O}{\|}}{C} - (X)_n - A$$

dans laquelle

X = CO, $C(R^1)(R^2)$ ou $C(R^3)(OR^4)$, où $R^1$, $R^2$, $R^3$ et $R^4$ représentent H ou des restes d'hydrocarbures,

n = 0 ou 1,

A = des restes d'hydrocarbures éventuellement substitués qui peuvent être reliés entre eux et, pour n = 1 et X = $C(R^1)(R^2)$ et pour n = 0, des restes aromatiques,

et

b) au moins un agent réducteur,

caractérisé en ce que l'agent réducteur est un 1-aryl-2,5-dialkyl-pyrrole.

8. Matériau photopolymérisable selon la revendication 7, caractérisé en ce que le photosensibilisateur est le benzile ou la camphoquinone.

9. Matériau photopolymérisable selon la revendication 8, caractérisé en ce que le photosensiblisateur consiste en camphoquinone et un dialkylacétal du benzile.

10. Matériau photopolymérisable selon la revendication 9, caractérisé en ce qu'il contient comme composé vinylique un ester d'acide acrylique et/ou un ester d'acide méthacrylique.